# EUROPEAN PATENT APPLICATION

(11) **EP 1 371 382 A1**
(43) Date of publication of application: **17.12.2003**
(21) Application number: 02425366.8
(22) Date of filing: 04.06.2002
(51) Int. Cl.: A61M 5/32

(54) **Safety syringe having a protective sleeve**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A disposable syringe (100) comprises a syringe body (1) hollow on the inside, a plunger (4) sliding inside the syringe body (1) and provided at the rear with a manually operable stem (41), an injection needle (2) that can be attached to the fore end (13) of the syringe body (1), by means of a needle-carrier (3), a sleeve (5) mounted slidably over the syringe body (1) to pass from a retracted position of use of the syringe to a forward position of safety in which it covers the needle (2), spring means (7) disposed under compression between the sleeve (5) and the syringe body (1), locking means (56, 58, 12) provided in the rear part of the sleeve (5) and of the syringe body (1) in mutual engagement to lock the sleeve in a retracted position of use, operating means (44) disposed in the stem (41) to release the locking means when the plunger (4) reaches the end of the injection stroke, and guide means (60) disposed in the sleeve and cooperating with complementary guide means (17) provided in the syringe body (1) to guide the axial movement of the sleeve (5) with respect to the syringe body.

## Description

The present invention refers to a simplified disposable safety syringe provided with an outer sleeve that is automatically guided into a position of safety, once the injection has been completed.

As is known, a syringe generally comprises a cylindrical body open at the front and rear to receive a plunger. A needle, hollow on the inside, is mounted at the head of the syringe body. The liquid contained in a vial is drawn through the needle into the syringe body by retracting the plunger. The liquid contained in the syringe body is injected through the needle into the patient's body by pressing on the plunger.

Because of hygiene rules and to avoid transmission of infectious diseases, syringes must generally be used only once and then discarded. For this reason, there is a growing demand on the market for disposable syringes that are able to prevent further use thereof.

Moreover, syringes generally present drawbacks from the point of view of safety. In fact, once the syringe has been used, the needle remains exposed at the fore end of the syringe body, with the risk of accidental injury or needle sticks.

Patent application PCT WO 99/37345 discloses a disposable safety syringe which provides a needle-covering sleeve mounted axially on the syringe body and slidable from a retracted position, in which it leaves the needle exposed to allow injection, to a forward position in which it completely covers the needle, preventing re-use of the syringe and acting as a guard against accidental needle sticks.

Once the injection has been carried out, the sleeve is automatically brought into the forward safety position, by means of an automatic mechanism and without any intervention by the user. However, this solution holds a certain complexity due to the presence of various additional elements for operation of the automatic mechanism. Furthermore, the telescopic movement of the sleeve on the syringe body is not guided, with the result of possible jamming and malfunctioning of the safety device of the syringe.

The object of the present invention is to eliminate the drawbacks of the prior art, providing a disposable safety syringe that comprises a reduced number of elements and is therefore economical and simple to make.

Another object of the present invention is to provide such a disposable syringe that is versatile, practical and simple for the user to use.

Another object of the present invention is to provide such a disposable syringe that is able to prevent further attempts at use.

Yet another object of the present invention is to provide such a disposable syringe that is extremely safe and able to prevent accidental injuries after use thereof.

These objects are achieved in accordance with the invention with the characteristics listed in appended independent claim 1.

Advantageous embodiments of the invention are apparent from the dependent claims.

The disposable syringe according to the invention comprises a syringe body hollow on the inside and open at the front and rear, a plunger sliding inside the syringe body with an injection stroke extending from a retracted position of filling to a forward position of emptying of the syringe, and an injection needle that can be attached to the fore end of the syringe body, by means of a needle-carrier. The plunger is provided at the rear with a stem that can be operated manually and brought out of the syringe body through the rear end thereof.

The syringe further comprises a sleeve mounted slidably over the syringe body, to pass from a retracted position of use of the syringe to a forward position of safety, in which it covers the needle. Spring means are disposed under compression between the sleeve and the syringe body to urge the axial movement of the sleeve into the forward position of safety with respect to the syringe body.

The sleeve is locked in the position of use by means of locking means, in reciprocal engagement, provided in the rear part of the sleeve and of the syringe body. In the rear part of the stem, on the other hand, operating means are provided to release said locking means when the plunger reaches the end of the injection stroke, so as to allow the action of the spring means that automatically generate the axial movement of the sleeve with respect to the syringe body.

The peculiarity of the disposable syringe according to the invention is represented by the fact that said sleeve comprises guide means cooperating with complementary guide means provided in the syringe body to guide the axial movement of the sleeve with respect to the syringe body.

The advantages of the disposable syringe according to the invention are evident. In fact, once the injection has been completed, the sleeve is automatically guided in its telescopic movement on the syringe body, so as to cover the needle. In this manner, the needle is in a position of safety and thus accidental needle sticks and any attempts at reuse of the syringe are avoided.

Moreover, compared with a conventional syringe, said syringe has only two additional elements, that is to say the sleeve and the spring, therefore is extremely cheap and simple to make.

Further characteristics of the invention will be made clearer by the detailed description that follows, referring to purely exemplary and therefore non-limiting embodiments thereof, illustrated in the appended drawings, wherein:
Figure 1 is an exploded side view, illustrating the disposable safety syringe according to the invention;
Figure 2 is a cross sectional view of the syringe body taken along the sectional plane II-II of Figure 1;
Figure 3 is a cross sectional view of the syringe body taken along the sectional plane III-III of Figure 1;
Figure 4 is a side view of the syringe of Figure 1, assembled and without a needle;
Figure 5 is an axial sectional view, taken along the sectional plane V-V of Figure 4, wherein the piston stem has not been sectioned;
Figure 6 is a side view of the syringe according to the invention, assembled with the needle mounted at the head and ready for use;
Figure 7 is a side view of the syringe, according to the invention, in a position of safety.

The disposable safety syringe according to the invention, designated as a whole with reference numeral 100, is described with the aid of the figures.

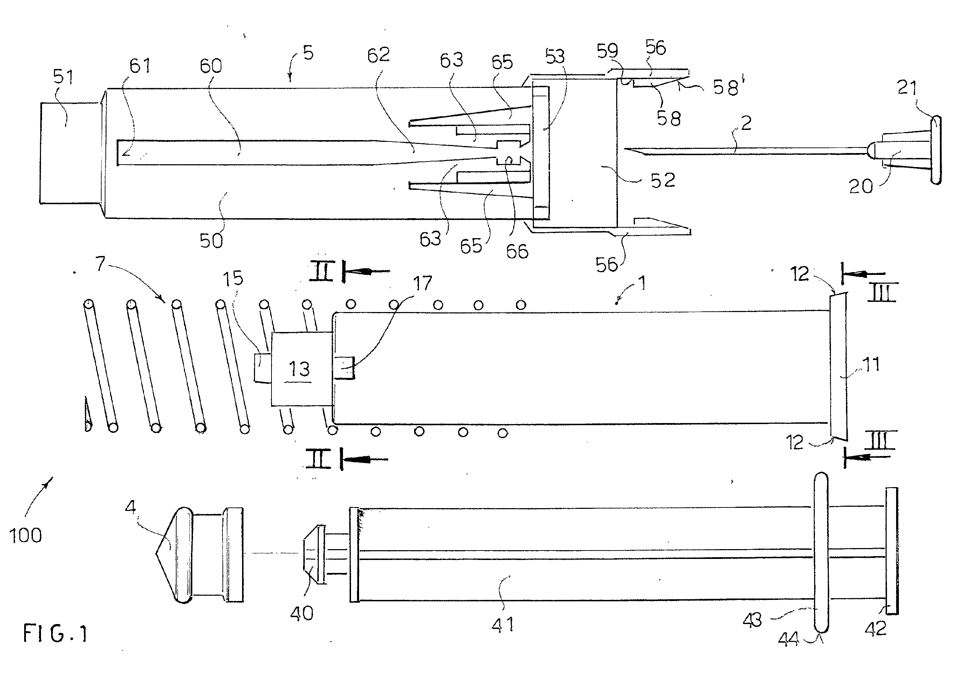

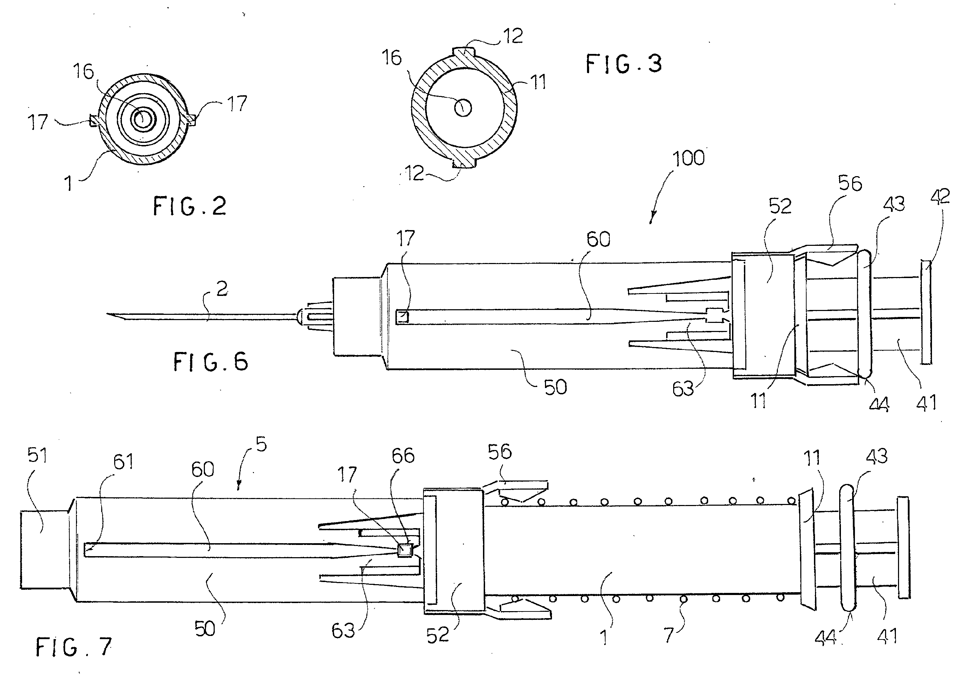

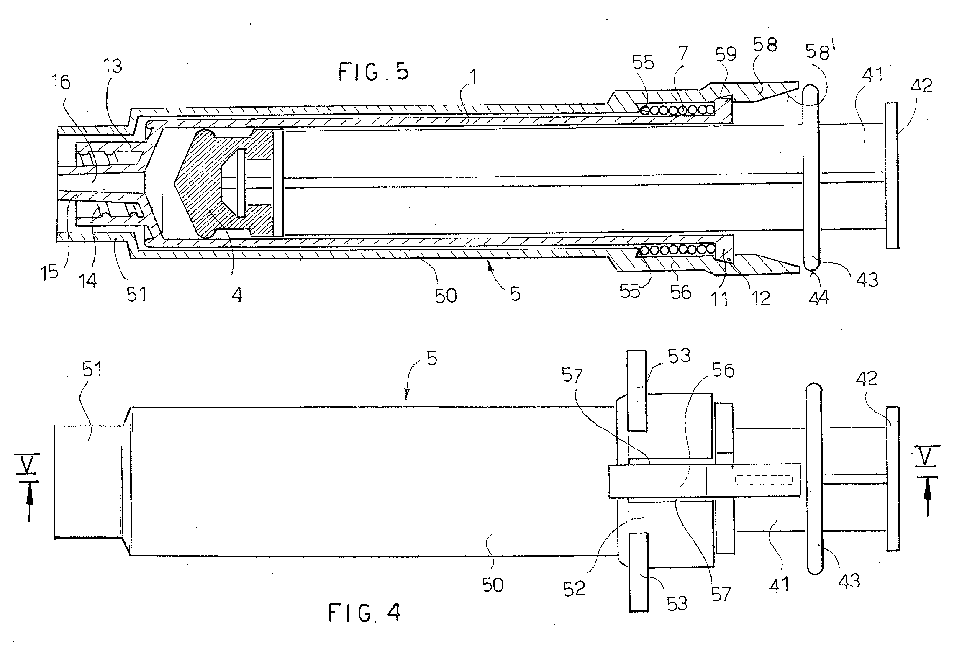

With reference in particular for now to Figure 1, the syringe 100 comprises a cylindrical body 1, hollow on the inside, defining a cylindrical chamber. The rear end of the body 1 is outwardly open and has an annular collar 11 protruding radially outward. As shown also in Figure 3, two tongues or nibs 12, which protrude radially outward, are disposed in diametrically opposite positions on the collar 11.

The front end of the body 1 ends with an outwardly open head 13, with a smaller diameter than the body 1. As shown in Figure 5, the head 13 has an inside thread 14.

Inside the head 13 a tang 15 with a smaller diameter than the head is disposed axially. The tang 15 has a truncated conical shape, hollow on the inside, having an axial hole 16 communicating with the inside of the body 1 and open toward the outside.

At the front end of the body 1, behind the head 13, as also shown in Figure 2, are two nibs or protrusions 17 disposed in diametrically opposite positions and offset by about 90° with respect to the two nibs 12 provided in the collar of the rear end of the syringe body 1.

A needle 2 is supported by a cylindrical or truncated conical support 20, hollow on the inside, having an axial chamber able to receive the tang 15 of the head 13 of the syringe body. The support 20 of the needle has at its free end a collar with tongues or an outside thread 21 able to engage in the inside thread 14 of the head 13 of the syringe body.

A plunger 4 can slide sealingly inside the chamber 10 of the syringe body 1. The plunger 4 is mounted on the head 40 of a stem 41 having a cross-shaped cross section. The stem 41 ends at the rear with a disc-shaped flange 42 that provides an abutment surface for the user's finger during the injection. For this purpose the rear surface of the flange 42 may have a knurled surface.

Near the rear flange 42, around the stem 41, an operating flange 43 that protrudes radially outward is provided. The operating flange 43 has a substantially elliptical shape and has a rounded side surface 44. The operating flange 43 can be made in a single body with the rear flange 42 of the stem 41.

A safety device of the syringe according to the invention is designated with reference numeral 5 and takes the form of a sleeve with a substantially cylindrical body 50, hollow on the inside, having an axial chamber open at the front and rear. The sleeve 5 has a front part 51 with a smaller diameter and a rear part 52 with a larger diameter with respect to the central part 50 of the body. The inside diameter of the central part 50 of the body of the sleeve is slightly greater than the outside diameter of the syringe body 1, so that the sleeve 5 can slide axially on the syringe body.

As shown better in Figure 4, between the central part 50 and the rear part 52 of the body of the sleeve there are provided two rigid tongues 53 which protrude radially outward, in diametrically opposite directions, to form a resting surface for the user's fingers. Again between the central part 50 and the rear part 52 of the body of the sleeve, as shown in Figure 5, an annular abutment surface 55 that protrudes radially inward is generated on the inside.

In the rear part 52 of the body of the sleeve two flexible longitudinal tongues 56 disposed in diametrically opposite positions are provided. As shown in Figure 4, each tongue 56 is defined by two longitudinal parallel notches 57 formed on the rear part 52 of the body of the sleeve, so as to be able to bend outward with respect to the body of the sleeve.

Each longitudinal tongue 56 has an inwardly protruding part 58 which defines a recessed seat 59 suitable to receive the radial tongues 12 of the collar 11 of the syringe body 1. Moreover, each tongue 56 protrudes rearward with respect to the rear edge of the rear part 52 of the sleeve and each protrusion 58 has, in its rear end, a substantially tapered part 58', able to cooperate with the rounded surface 44 of the operating flange 43 of the stem 41.

In the central part 50 of the body of the sleeve, two longitudinal slots 60 disposed in diametrically opposite positions, able to receive the nibs 17 of the syringe body 1 are formed. Each longitudinal slot 60 has a front abutment and end-of-stroke surface 61 and a rear narrowing 62.

Two flexible tongues 63 disposed opposite each other extend in the rear narrowing 62 of each longitudinal slot 60. Each flexible tongue 63 is defined by a longitudinal notch 65 made in the central part 50 of the body of the sleeve, parallel to the longitudinal slot 60. In this manner the flexible tongues 63, which normally occupy part of the longitudinal slot 60 generating the narrowing 62 of said slot, can bend in the notch 65 eliminating the narrowing 62 of said slot.

Each tongue 63 comprises a recessed seat 66 facing toward the narrowing 62 of the guide slot 60.

Lastly the syringe 100 comprises a coil spring 7, designed to be housed in the rear part 52 of the body of the sleeve and able to allow the syringe body 1 to pass on the inside thereof.

Assembly of the syringe 100 according to the invention is now illustrated, purely by way of example.

The plunger 4 is mounted in the head 40 of the stem 41 and inserted inside the syringe body 1. The spring 7 is inserted from the front part on to the syringe body 1, until one end of the spring 7 abuts against the collar 11 of the syringe body.

At this point the sleeve 5 is inserted from the rear on to the syringe body. During insertion the nibs 17 of the syringe body enter into the guide slots 60 of the sleeve 5 and the sleeve 5 is guided in its axial movement, compressing the spring 7 between the annular abutment surface 55 of the sleeve and the collar 11 of the syringe body. At the same time the longitudinal tongues 56 of the rear part 52 of the body of the sleeve bend outward, until the nibs 12 of the collar 11 of the syringe body enter the seats 59 of the tongues 56.

In this situation, as shown in Figure 6, the nibs 17 of the syringe body abut against the respective front abutment surfaces 61 of the longitudinal slots 60 of the sleeve and the nibs 12 of the collar of the syringe body are engaged inside the respective seats 59 of the longitudinal tongues 56 of the rear part of the sleeve. In this manner the sleeve 5 is made integral with the syringe body and any axial movement of the sleeve 5 with respect to the syringe body 1 is prevented. It should further be noted that in such a situation, the spring 7 is compressed with one end abutting against the collar 11 of the syringe body and the other end abutting against the annular abutment surface 55 of the rear part of the sleeve.

Lastly, the needle 2 is mounted in the head of the syringe body, by screwing the outside thread 21 of the needle support 20 into the inside thread 15 of the syringe body.

Operation of the syringe 100 according to the invention will now be described.

As shown in Figure 6, when the plunger 4 reaches the end of the injection stroke inside the chamber of the syringe body, the operating flange 43 of the stem 41 comes into contact with the longitudinal flexible tongues 56 of the rear part of the sleeve. Precisely, the rounded surface 44 of the operating flange of the stem cooperates with the tapered surface 58' of the tongues of the rear part of the sleeve, causing outward bending of said tongues 56.

As a result, the nibs 12 of the collar of the syringe body disengage from the seats 59 of the tongues 56. Consequently, axial movement of the sleeve 5 with respect to the syringe body 1 is no longer prevented. Thus, through the action of the spring 7 which is released, the sleeve 5 moves axially forward with respect to the syringe body 1 and/or the syringe body 1 moves axially rearward with respect to the sleeve 5.

During said axial movement, the sleeve 5 is guided by the nibs 17 of the syringe body inside the longitudinal slots 60 of the sleeve. When the nibs 17 of the syringe body reach the rear part of the respective slots 60, said nibs 17 push the flexible tongues 63 which bend in the respective notches 65.

At the end of the stroke of the sleeve 5, as shown in Figure 7, the nibs 17 of the syringe body are caught and held firmly between the seats 66 of a pair of opposite facing tongues 65. In this situation the needle 2 is protected by the sleeve 5 which is in its forward position of safety. It should be noted that in said situation, the flexible tongues 63 of the sleeve return to their initial configuration and thus the nibs 17 of the syringe body are locked between the seats 66 of the flexible tongues 65. In this manner, any axial movement of the sleeve with respect to the syringe body is prevented.

Numerous variations and changes of detail, within the reach of a person skilled in the art, can be made to the present embodiment of the invention, without departing from the scope of the invention, as set forth in the appended claims.

## Claims

1. A disposable syringe (100) comprising:
- a syringe body (1) hollow on the inside and open at the front and rear,
- a plunger (4) slidable inside the syringe body (1) with an injection stroke extending from a retracted position of filling to a forward position of emptying of the syringe, said plunger (4) being provided at the rear with a stem (41) that can be operated manually and brought out of the syringe body through the rear end (42) thereof,
- an injection needle (2) attachable to the front end (13, 15) of the syringe body (1),
- a sleeve (5) slidably mounted over said syringe body (1), to pass from a retracted position of use of the syringe to a forward position of safety, wherein it covers said needle (2), and
- spring means (7) disposed under compression between said sleeve (5) and said syringe body (1) to urge the axial movement of the sleeve (5) with respect to the syringe body,
**characterised in that** it further comprises
- locking means (56, 58, 12) provided in the rear part of the sleeve (5) and in the rear part of the syringe body (1), in reciprocal engagement, to keep the sleeve locked in the retracted position of use against the action of the spring means (7),
- operating means (44) disposed in said stem (41) to release said locking means, when the plunger (4) reaches the end of the injection stroke, so as to allow axial movement of the sleeve into the safety position, thanks to the action of the spring means, and
- guide means (60) provided in said sleeve (5) and cooperating with complementary guide means (17) formed in the syringe body (1) to guide the axial movement of the sleeve (5) with respect to the syringe body (1).

2. A syringe according to claim 1, **characterised in that** said guide means of the sleeve (5) comprise a pair of longitudinal through slots (60) disposed in diametrically opposite positions in the central part of said sleeve and said guide means of the syringe body (1) comprise a pair of teeth or nibs (17), disposed in diametrically opposite positions on the syringe body (1), able to engage in a sliding coupling relationship within the respective longitudinal slots (60).

3. A syringe according to claim 2, **characterised in that** said longitudinal slot (60) of the sleeve comprises an abutment and front end-of-stroke surface (61) and a locking and rear end-of-stroke seat (66).

4. A syringe according to claim 3, **characterised in that** provided in said longitudinal slot (60) is a pair of opposite facing flexible tongues (63) obtained by means of respective notches (65) in the body of the sleeve, parallel to the longitudinal slot (60), so that said flexible tongues (63) give rise to a narrowing (62) of said longitudinal slot (60).

5. A syringe according to claim 3 or 4, **characterised in that** each flexible tongue (63) has a recessed seat (66) facing toward said longitudinal slot (60).

6. A syringe according to any one of the preceding claims, **characterised in that** said locking means comprise a pair of tongues or nibs (12) protruding radially outward, in diametrically opposite positions, in the rear part of the syringe body and a pair of flexible longitudinal tongues (56) formed in diametrically opposite positions in the rear part (52) of said sleeve (5), said flexible tongues (56) having respective seats (59) able to receive said respective nibs (12) in a locking relationship.

7. A syringe according to claim 6, **characterised in that** said flexible tongues (56) have a rear part protruding rearward with respect to the rear edge of the sleeve and said operating means of the stem comprise an operating flange (43) disposed in the rear part of the stem (41) cooperating with said rear part of the flexible tongues (56) of the sleeve so as to give rise to outward bending of the said flexible tongues (56) and release of said nibs (12) from the seats (59) of the flexible tongues.

8. A syringe according to claim 7, **characterised in that** the rear end of said flexible tongues (56) has a tapered inner surface (58') able to cooperate with a complementary rounded surface (44) provided in the lateral part of said operating flange (43).

9. A syringe according to any one of the preceding claims, **characterised in that** said spring means comprise a coil spring (7) disposed around the syringe body (1) with one end abutting against a collar (11) protruding outward from the rear part of the syringe body and the other end abutting against an annular abutment surface (55) provided within the rear part (52) of said sleeve.

10. A syringe according to any one of the preceding claims, **characterised in that** said injection needle (2) comprises a support (20) hollow on the inside and provided with an outside thread (21) and said front end of the syringe body (1) comprises a cylindrical head (13) provided with an inside thread (14) complementary to the outside thread (21) of the needle carrier and a substantially conical tang (15), disposed axially inside the head, to engage inside the needle support (20).
